(19) Europäisches Patentamt / European Patent Office / Office européen des brevets

(11) EP 2 913 071 B1

(12) # EUROPÄISCHE PATENTSCHRIFT

(45) Veröffentlichungstag und Bekanntmachung des Hinweises auf die Patenterteilung:
**26.12.2018 Patentblatt 2018/52**

(51) Int Cl.:
***A61M 1/14*** *(2006.01)* ***A61M 1/34*** *(2006.01)*
***A61M 1/36*** *(2006.01)*

(21) Anmeldenummer: **15154738.7**

(22) Anmeldetag: **11.02.2015**

(54) **Vorrichtung zur Erkennung eines Fehlers im extrakorporalen Blutkreislauf**

Device for detecting an error in extracorporeal blood circulation

Dispositif de reconnaissance d'une erreur dans une circulation sanguine extra-corporelle

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.02.2014 DE 102014102730**

(43) Veröffentlichungstag der Anmeldung:
**02.09.2015 Patentblatt 2015/36**

(73) Patentinhaber: **B. Braun Avitum AG**
**34212 Melsungen (DE)**

(72) Erfinder:
- **Strohhöfer, Dr. Christof**
  **34123 Kassel (DE)**
- **Schreiber, Jens**
  **34121 Kassel (DE)**
- **Steger, Jennifer**
  **34212 Melsungen (DE)**

(74) Vertreter: **Winter, Brandl, Fürniss, Hübner, Röss, Kaiser, Polte - Partnerschaft mbB**
**Patent- und Rechtsanwaltskanzlei**
**Alois-Steinecker-Strasse 22**
**85354 Freising (DE)**

(56) Entgegenhaltungen:
**EP-B1- 1 584 339** **WO-A1-00/66197**
**DE-B3-102012 024 341**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 913 071 B1

Printed by Jouve, 75001 PARIS (FR)

## Beschreibung

Die Erfindung betrifft eine Vorrichtung bzw. ein System zur Erkennung eines Fehlers im extrakorporalen Blutkreislauf einer Vorrichtung zur extrakorporalen Blutbehandlung. Ein Beispiel für einen solchen Fehler ist eine venöse Nadeldiskonnektion (VND).

Zur extrakorporalen Blutbehandlung mittels einer Vorrichtung wie einer Dialysemaschine wird Blut eines Patienten beispielsweise im Zuge einer Hämodialyse, Hämofiltration oder Hämodiafiltration über einen extrakorporalen Blutkreislauf geleitet. Um Zugang zum intrakorporalen Blutgefäßsystem des Patienten zu erhalten, werden in der Regel arteriovenöse Fisteln, Shunts oder auch Gefäßimplantate verwendet. Die Verbindung des extrakorporalen Blutkreislaufs mit dem Patienten erfolgt üblicherweise über Katheter oder Kanülen bzw. Nadeln, z.B. Dialysekanülen oder -nadeln, mit denen beispielsweise eine Fistel, ein Shunt oder ein Gefäßimplantat punktiert wird.

Im Rahmen einer solchen Blutbehandlung können Fehler im extrakorporalen Blutkreislauf auftreten, die kurz als FEB bezeichnet werden. Ein Beispiel für einen FEB ist eine Nadeldiskonnektion. Dabei ist ein Zugang zum Blutkreislauf des Patienten gestört, beispielsweise wenn eine Nadel oder Kanüle verrutscht und der extrakorporale Kreislauf nicht mehr ordnungsgemäß mit dem intrakorporalen Blutkreislauf, auch Patientenblutkreislauf, verbunden ist. Dies kann insbesondere bei einer Diskonnektion eines venösen Zugangs zum Patientenblutkreis problematisch sein. Wird diese nicht rechtzeitig erkannt, wird über einen arteriellen Zugang weiterhin Blut des Patienten entnommen, jedoch nach der extrakorporalen Blutbehandlung dem Patienten nicht wieder oder nur in unzureichender Menge zugeführt. Bei üblichen Blutflussraten von etwa 300 bis 400 ml/min kann sich so innerhalb weniger Minuten eine lebensgefährliche Situation ergeben.

Die Erkennung von Fehlern im extrakorporalen Blutkreislauf, insbesondere die Erkennung einer venösen Nadeldiskonnektion, ist ein schwerwiegendes Problem bei medizinischen Behandlungen unter Nutzung eines extrakorporalen Blutkreislaufs wie beispielsweise der Hämodialyse. Allein in den USA sterben pro Jahr beispielsweise etwa einhundert Menschen an den Folgen einer venösen Nadeldiskonnektion (Hurst, Jane, "Venous Needle Dislodgement - A Universal Concern". European Nephrology).

Bekannte Verfahren und Systeme zum Erkennen eines Fehlers im extrakorporalen Blutkreislauf, insbesondere zu dessen Erkennung vor einem Entstehen schwerwiegender Folgen für den Patienten, weisen in nachteiliger Weise Schwächen auf. Außerdem sind bekannte Verfahren und Systeme meist anfällig für Fehlalarme. Häufige Fehlarme sind ebenso kritisch wie nicht erkannte FEBs, da sie zur Abstumpfung des Personals führen.

Es sind Verfahren zur Erkennung eines Fehlers im extrakorporalen Blutkreislauf (FEB) bekannt, die auf einer Analyse des venösen (und/oder arteriellen) Drucks beruhen. Die EP 1 584 339 B1 offenbart zum Beispiel ein Verfahren zum Erkennen einer Nadeldiskonnektion auf der Basis einer Messung von arteriellen und venösen Drücken unter Summen- und Differenzbildung.

Die US 7,648,474 B2 offenbart eine Vorrichtung, die zum Ermitteln einer Nadeldiskonnektion arterielle und venöse Druckwerte überwacht.

Die EP 1 815 878 B1 offenbart eine Blutreinigungsvorrichtung mit einer Einrichtung zum Messen des venösen Blutdruckes eines Patienten und einer Einrichtung zum Überwachen des venösen Blutdruckes, wobei durch Vergleichen eines vorbestimmten Alarmschwellenwertes mit einem gemessenen Druck oder mit einem Druck, dessen Messung vorhergesagt wird, ein Alarm aktiviert wird, wobei die Einrichtung zum Überwachen des venösen Blutdruckes den vorbestimmten Alarmschwellenwert bei einer vorbestimmten Zeitdauer aktualisiert.

Aus der DE 10 2012 024 341 B3 sind eine Vorrichtung und ein Verfahren zum Überwachen eines extrakorporalen Blutkreislaufs zur Erkennung von Luftblasen beschrieben, bei dem eine Überwachung eines Unterdrucks im extrakorporalen Blutkreislauf stromab einer Blutpumpe durchgeführt wird, um zwischen der Entstehung von Mikroblasen, die auf eine Kavitation zurückzuführen sind, und einem Lufteintrag, der nicht durch eine Kavitation bedingt ist, unterscheiden zu können. Wenn der Unterdruck oberhalb eines vorgegebenen Grenzwerts liegt und im extrakorporalen Blutkreislauf Mikroblasen erkannt werden, wird darauf geschlossen, dass die Mikroblasen durch Kavitation erzeugt werden.

Ein Nachteil bekannter Verfahren ist, dass diese nur einen begrenzten Bereich von Druckcharakteristika von Fehlern im extrakorporalen Blutkreislauf abdecken. Grund ist, dass im extrakorporalen Blutkreislauf auftretende Fehler in der Regel keine eindeutige Charakteristik, sondern ein breites Band möglicher Charakteristika aufweisen. Ein einzelnes auf der Analyse von Sensorwerten beruhendes Verfahren oder Überwachungssystem ist nicht in der Lage, dieses Band möglicher Charakteristika vollständig abzudecken und gleichzeitig andere mögliche Ursachen (neben einem FEB) für Sensorschwankungen auszuschließen.

Ausgehend vom vorbeschriebenen Stand der Technik liegt der Erfindung die Aufgabe zugrunde, ein verbessertes System zur Erkennung eines Fehlers im extrakorporalen Blutkreislauf bzw. zur Überwachung der Unversehrtheit des extrakorporalen Blutkreislaufs bei einer extrakorporalen Blutbehandlung zu schaffen. Es soll eine Vielzahl von Fehlern im extrakorporalen Blutkreislauf mit hoher Sicherheit und Verlässlichkeit erfasst werden können. Des Weiteren sollen Fehlalarmierungen vermieden werden können. Die Überwachung soll vorzugsweise anhand von Sensorwerten erfolgen und lebensbedrohliche Situationen z.B. aufgrund von Blutverlust eines Patienten sollen sicher vermieden werden können.

Diese Aufgabe wird erfindungsgemäß durch ein Fehlererkennungssystem gemäß Anspruch 1 gelöst. Vorteilhafte Ausgestaltungen der Erfindung sind Gegenstand der Unteransprüche.

Das System erlaubt eine Erkennung eines Fehlers im extrakorporalen Blutkreislauf einer Vorrichtung zur extrakorporalen Blutbehandlung (nachfolgend "Blutbehandlungsmaschine"), z.B. einer Dialysemaschine. Dabei kann zumindest ein Zustandsparameter, erfasst werden, ein erstes Bewertungskriterium zur Erkennung eines Fehlers im extrakorporalen Blutkreislauf (FEB) bestimmt werden, unter Verwendung des ersten Bewertungskriteriums eine Entscheidung hinsichtlich des Vorliegens eines Fehlers im extrakorporalen Blutkreislauf getroffen und ein erstes Fehlersignal generiert sowie der erfasste Zustandsparameter überwacht wird, wobei zumindest ein weiteres Bewertungskriterium bestimmt wird, unter Verwendung des einen weiteren Bewertungskriteriums eine Entscheidung hinsichtlich des Vorliegens eines Fehlers im extrakorporalen Blutkreislauf getroffen und zumindest ein weiteres Fehlersignal generiert wird, das erste Fehlersignal und das zumindest eine weitere Fehlersignal zu einem kombinierten Fehlersignal kombiniert werden und vorzugsweise ein Alarm ausgelöst wird, wenn das kombinierte Fehlersignal einen vorbestimmten Grenzwert (im positiven wie im negativen Sinne) überschreitet.

Das System zur Erkennung eines Fehlers im extrakorporalen Blutkreislauf einer extrakorporalen Blutbehandlungsmaschine, z.B. einer Dialysemaschine, weist zumindest einen Sensor zur Erfassung zumindest eines Zustandsparameters, eine Bewertungseinheit zur Bestimmung eines ersten Bewertungskriteriums zur Erkennung eines Fehlers im extrakorporalen Blutkreislauf (FEB), eine Überwachungseinheit zum Treffen einer Entscheidung hinsichtlich des Vorliegens eines Fehlers im extrakorporalen Blutkreislauf und zum Generieren eines ersten Fehlersignals sowie zur Überwachung des erfassten Zustandsparameters, jeweils unter Verwendung des ersten Bewertungskriteriums, wobei das System zumindest eine weitere Bewertungseinheit aufweist, um zumindest ein weiteres Bewertungskriterium zu bestimmen, das System zumindest eine weitere Überwachungseinheit aufweist, um unter Verwendung des zumindest einen weiteren Bewertungskriteriums eine Entscheidung hinsichtlich des Vorliegens eines Fehlers im extrakorporalen Blutkreislauf zu treffen und zumindest ein weiteres Fehlersignal zu generieren, und das System eine Kombinierungseinheit aufweist, um das erste Fehlersignal und das zumindest eine weitere Fehlersignal zu einem kombinierten Fehlersignal zu kombinieren.

Die extrakorporale Blutbehandlungsmaschine, vorzugsweise Dialysemaschine kann der Durchführung von chronischen Blutreinigungstherapien im weitesten Sinne, wie z.B. Hämodialyse, Hämofiltration oder Hämodiafiltration, dienen. Mit der Erfindung werden beliebige Fehler im extrakorporalen Blutkreislauf, kurz als FEB bezeichnet, erfasst/ermittelt. Ein solcher FEB kann zum Beispiel in Form einer Verstopfung bzw. eines Verschlusses oder einer Verengung des extrakorporalen Blutkreislaufs oder in Form eines Lecks bestehen. Ein Beispiel für einen FEB ist eine Nadeldiskonnektion, insbesondere eine venöse Nadeldiskonnektion (kurz VND).

Der Begriff Zustandsparameter ist allgemein zu verstehen und kann insbesondere einen hämodynamischen Zustandsparameter umfassen. Der (wenigstens eine) Zustandsparameter kann ein Parameter eines Patienten, ein Parameter des extrakorporalen Blutkreislaufs oder ein Parameter von anderen Einheiten der Dialysemaschine sein. Er kann vor und/oder während und/oder nach einer Behandlung erfasst werden, sowohl kontinuierlich als auch diskontinuierlich. Vorzugsweise wird der (wenigstens eine) Zustandsparameter mittels eines geeigneten Sensors oder einer geeigneten Sensoreinheit erfasst. Im Rahmen der Erfindung können ein einziger oder mehrere Zustandsparameter erfasst werden. Beispiele für den wenigstens einen Zustandsparameter sind der venöse Blutdruck und/oder der arterielle Blutdruck, oder die Blutströmungsrate oder dergleichen strömungsmechanische Parameter. Weitere Zustandsparameter könnten auch sein: Hämatokrit, Dichte, chemische Zusammensetzung des Plasmas, Konzentration einzelner Stoffe im Blut jeweils einzeln oder in Kombination als Konzentrationsverhältnis.

Nach der Erfindung kann die Bestimmung von Bewertungskriterien für einen oder mehrere erfasste Zustandsparameter vorzugsweise mittels einer Bewertungseinheit oder mehrerer Bewertungseinheiten erfolgen. Die Überwachung der erfassten Zustandsparameter unter Verwendung des ersten Bewertungskriteriums bzw. der weiteren Bewertungskriterien kann nach der Erfindung insbesondere mittels einer Überwachungseinheit oder mittels mehrerer Überwachungseinheiten erfolgen. Die Kombination der Fehlersignale kann nach der Erfindung insbesondere mittels gewichteter oder ungewichteter Fallanalysen, Fuzzy-Modellen, neuronaler Netze, SVR’s oder physikalischer oder mathematischer Modelle, die z.B. von der Temperatur oder von anderen physikalischen Größen abhängig sind, erfolgen.

Anders ausgedrückt kommen zur Überwachung der Dialysemaschine drei unterschiedliche Einheiten zum Einsatz, nämlich zum ersten eine Bewertungseinheit oder mehrere Bewertungseinheiten, die ein Bewertungskriterium zur Erkennung eines FEB bestimmt bzw. bestimmen, zum zweiten eine Überwachungseinheit oder mehrere Überwachungseinheiten, die aus den bestimmten Bewertungskriterien der Bewertungseinheit einen FEB ermittelt bzw. ermitteln, und zum dritten eine Kombinierungseinheit oder mehrere Kombinierungseinheiten, mit der bzw. denen mehrere Überwachungseinheiten und damit indirekt auch Bewertungseinheiten kombiniert werden. Eine, mehrere oder alle genannten drei Einheiten können jeweils eine Speichereinheit, eine Recheneinheit, eine Energieversorgung und eine Datenleitung aufweisen.

In den Bewertungseinheiten werden Bewertungskriterien bestimmt. Das erste Bewertungskriterium und das zumindest eine weitere Bewertungskriterium können identisch oder verschieden sein. Geeignete Bewertungskriterien können beispielsweise durch polynominale Regression und exponentiell gewichtetes gleitendes Mittel bestimmt werden.

Die Überwachungseinheit generiert ein Fehlersignal, das der Kombinierungseinheit zugeführt wird. Insbesondere kann eine Überwachungseinheit ein Fehlersignal generieren, das im Falle einer Erkennung eines Fehlers im extrakorporalen Blutkreislauf auf einen bestimmten ersten Wert gesetzt wird und für den Fall, dass kein FEB erkannt wird, auf einen anderen, zweiten Wert gesetzt wird.

Die Kombinierungseinheit wird genutzt, um beliebig viele Überwachungseinheiten und damit auch die von diesen generierten Fehlersignale zu einem kombinierten Fehlersignal zu kombinieren. Diese Kombination ist von entscheidendem Vorteil. Es wird nicht nur anhand eines bewerteten Fehlersignals auf das Vorliegen eines FEB geschlossen, wie aus dem Stand der Technik bekannt, sondern es wird eine Mehrzahl vorzugsweise unterschiedlich bewerteter Fehlersignale in der Kombinierungseinheit verarbeitet. Die Stärken und Schwächen der jeweiligen Bewertungseinheiten und der darin durchgeführten Bewertungen sind bekannt oder können ermittelt werden, und so kann durch gezielte Auswahl oder Verarbeitung der von den Überwachungseinheiten an die Kombinierungseinheit geleiteten Fehlersignale die Qualität der Fehlererkennung und -anzeige verbessert und Fehlalarme vermieden werden. Mit der erfindungsgemäßen Vorrichtung lässt sich insbesondere eine venöse Nadeldiskonnektion frühzeitig erkennen. Dadurch kann eine hohe Patientensicherheit bei einer Behandlung wie etwa einer Dialysebehandlung und auch eine hohe Funktionssicherheit gewährleistet werden.

Ein von einer Überwachungseinheit generiertes Fehlersignal kann binär sein, z.B. "0" für kein FEB oder Konnektion und "1" für FEB oder Diskonnektion. Alternativ kann ein Fehlersignal in nichtbinärer Form vorliegen, z.B. in Form von Werten in einem Bereich von 0 bis 1. Auch eine Kombination dieser Varianten liegt im Rahmen der Erfindung, z.B. können ein Fehlersignal einer Überwachungseinheit in binärer Form und ein Fehlersignal einer anderen Überwachungseinheit in nichtbinärer Form vorliegen. Die Nutzung von nichtbinären Fehlersignalen besitzt den Vorteil, dass im Rahmen einer Entscheidung, ob ein FEB vorliegt oder nicht, berücksichtigt werden kann, wie weit ein festgelegter Grenzwert für das Vorliegen eines FEB unter- bzw. überschritten wurde.

Bisher gibt es kein Verfahren, welches ermöglicht, verschiedene Erkennungsmethoden zur Erkennung von Fehlern im extrakorporalen Blutkreislauf, wie z.B. einer VND, zu kombinieren. Mit der vorliegenden Erfindung wird eine Kombination von verschiedenen Bewertungseinheiten zur Erkennung eines FEB ermöglicht. Darüber hinaus kann einzelnen Bewertungsverfahren ein gezielter Anteil zur Erkennung von Fehlern zugewiesen werden, die Bewertungsverfahren können also unterschiedlich gewichtet werden. Des Weiteren können einzelne Bewertungsverfahren zu bestimmten verschiedenen Zeitpunkten gestartet werden. Damit wird ein Detektionssystem in die Lage versetzt, die spezifischen Stärken jeder beinhalteten Bewertungseinheit, also jedes genutzten Bewertungsverfahrens, zu nutzen und dessen Schwächen durch Stärken einer anderen Bewertungseinheit zu kompensieren.

Das erste und/oder das zumindest eine weitere Fehlersignal werden einer Gewichtung unterzogen. Insbesondere können alle Fehlersignale gewichtet werden. Im Rahmen einer solchen Gewichtung wird ein Fehlersignal oder werden mehrere Fehlersignale mit einem Faktor (dem Gewicht) multipliziert. Die dabei verwendete Größe des Faktors, die Gewichtung der Fehlersignale, kann frei gewählt werden. Infolge der Gewichtung kann z.B. ein Fehlersignal, von dem man weiß, dass es in bestimmten Zeitabschnitten oder unter bestimmten Umständen fehleranfällig sein kann, mit einer geringen Gewichtung versehen werden, während ein anderes Fehlersignal, das weniger fehleranfällig ist, mit einer höheren Gewichtung versehen werden kann. Ein Grenzwert der Fehlersignale, insbesondere der gewichteten Fehlersignale, bei dessen Erreichen oder Überschreiten von einem FEB auszugehen ist, kann entsprechend der Gewichte bzw. des kombinierten Fehlersignals möglicherweise auf die Art des FEB's zurückgeschlossen werden.

Zum Beispiel kann bei einem Verfahren nach der Erfindung, bei dem drei binäre Fehlersignale erzeugt werden, für jedes Fehlersignal ein Faktor von 0,5 als Gewichtung gewählt werden. Ein Grenzwert, der für das Vorliegen eines FEB bestimmt ist, kann auf einen Wert von 1 gesetzt werden. In diesem beispielhaften Fall wird ein FEB-Alarm ausgelöst, sobald zwei der von den Überwachungseinheiten ausgegebenen Fehlersignale einen FEB detektiert haben. Bei derartigen Einstellungen wurden in Tests ca. 95% aller FEB's erkannt und die Anzahl der Fehlalarme um ca. 8% gegenüber den bestehenden Systemen verringert.

Das erste Fehlersignal und das zumindest eine weitere Fehlersignal werden kombiniert, indem die die gewichteten Fehlersignale, summiert werden. Die Fehlersignale können insbesondere in einer Recheneinheit aufaddiert werden. In dem Fall, dass diese Summe einen Grenzwert überschreitet, einen bestimmten Bereich verlässt und/oder in einem bestimmten Bereich liegt, kann dann vom Vorliegen eines FEB ausgegangen werden und vorzugsweise eine Alarmierung veranlasst werden. Die Fehlersignale der Überwachungseinheiten können außerdem mit anderen mathematischen Methoden und Modellen kombiniert werden. Dabei kann ein Modell auf die einzelnen Überwachungseinheiten angepasst werden. Mögliche andere Kombinationsvarianten stammen aus dem Bereich des maschinellen Lernens, wie zum Beispiel gewichtete oder ungewichtete Fallanalysen, Fuzzy-Modelle, neuronale Netze, SVR's oder physikalische oder mathematische Modelle, die z.B. von der Temperatur oder von anderen physikalischen Größen abhängig sind.

Weiterbildungen der Erfindung sind in den abhängigen Ansprüchen definiert.

Nach einer Ausführungsform der Erfindung kann eine Initialisierung durchgeführt werden. Dabei kann für zumindest einen erfassten Zustandsparameter eine Initialisierung durchgeführt werden, indem dem Zustandsparameter ein Initialwert zugewiesen wird und das Bewertungskriterium unter Verwendung des Initialwerts sowie von nach der Initialisierung erfassten Zustandsparametern bestimmt wird.

Nach einer anderen Ausführungsform können eine Überwachung eines erfassten Zustandsparameters und ein Feststellen eines Fehlers mittels des ersten Bewertungskriteriums zeitlich versetzt zu einer Überwachung eines erfassten Zustandsparameters und einem Feststellen eines Fehlers mittels des zumindest einen weiteren Bewertungskriteriums ablaufen oder beginnen. Ein solcher zeitlicher Versatz kann insbesondere nach einem Systemstart und/oder einer Initialisierung und/oder einer Reinitialisierung erfolgen.

Nach einer Ausführungsform kann zumindest eine Störgröße, insbesondere eine solche, die Sensorwerte in gleichem oder ähnlichem Maße wie ein FEB beeinflussen, erfasst werden. Diese kann bzw. können für eine Entscheidung hinsichtlich des Vorliegens eines Fehlers berücksichtigt werden. Störgrößen können insbesondere kontinuierlich und/oder mit beliebiger Abtastrate erfasst werden. Beispiele für eine Störgröße sind insbesondere eine Ultrafiltrationsrate, eine Dialysierflüssigkeitsflussrate, eine Blutflussrate, eine Pegelregulierung oder ein vorangegangener Alarm. Nach Erfassung einer Störgröße kann insbesondere mit Vorteil eine Reinitialisierung durchgeführt werden. Durch eine solche Erfassung von Störgrößen können in vorteilhafter Weise Fehlalarme vermieden werden. Durch eine erfolgreiche Reduzierung von Fehlalarmen kann mit besonderem Vorteil ein Abstumpfen von Betreuungspersonal gegenüber Alarmierungen verringert oder verhindert werden.

Das erfindungsgemäße System kann insbesondere Bestandteil einer Blutbehandlungsvorrichtung, beispielsweise einer Dialysevorrichtung, sein. Es kann insbesondere eine Anzeige- und/oder Alarmeinrichtung zur Erzeugung einer Anzeige oder eines Alarms im Falle einer Ausgabe eines kombinierten Fehlersignals aufweisen.

Weitere Vorteile und Merkmale der Erfindung ergeben sich aus der folgenden Erläuterung der Erfindung sowie von bevorzugten Ausführungsbeispielen unter Bezugnahme auf die Zeichnungen. Es zeigen:

Fig. 1 einen Blutkreislauf einer Dialysemaschine bei einer Hämodialyse,

Fig. 2 eine schematische Darstellung von Zustandsautomaten für eine Bewertungs- und eine Überwachungseinheit,

Fig. 3 eine schematische Darstellung einer ersten Ausführungsform eines erfindungsgemäßen Systems,

Fig. 4 eine schematische Darstellung einer zweiten Ausführungsform eines erfindungsgemäßen Systems,

Fig. 5 ein Diagramm mit der Darstellung des venösen Druckverlaufs eines Patienten zusammen mit mittels einer ersten Bewertungseinheit bestimmten Ober- und Untergrenzen als Bewertungskriterien, und

Fig. 6 ein Diagramm mit der Darstellung des venösen Druckverlaufs eines Patienten zusammen mit mittels einer zweiten Bewertungseinheit bestimmten Ober- und Untergrenzen als Bewertungskriterien.

Die Beschreibung der Erfindung anhand der Ausführungsbeispiele des erfindungsgemäßen Systems und erfolgt mit Bezug auf sogenannte venöse Nadeldiskonnektionen oder Verlagerungen (VND, "Venous Needle Dislodgement"). Diese Bezugnahme auf eine VND ist nicht beschränkend und die Erfindung und die beschriebenen Ausführungsbeispiele können der Erfassung beliebiger Fehler im extrakorporalen Blutkreislauf einer Blutbehandlungsvorrichtung dienen.

Aufgrund von Bewegung des Patienten oder von unzureichender Befestigung einer Nadel/Kanüle an einem Patienten oder einer Blutleitung eines extrakorporalen Blutkreislaufes mit einer Nadel/Kanüle als Verbindung zwischen dem Patientenblutkreislauf und dem extrakorporalen Blutkreislauf einer Dialysemaschine kann es zu einem vollständigen oder teilweisen Lösen der Nadel, einer Diskonnektion, kommen. Dies ist insbesondere bei einer venösen Nadel problematisch, da über diese unter einem entsprechenden Druck stehendes Blut zum Patienten zurückgeführt wird. Dies kann die Gefahr einer Diskonnektion zusätzlich erhöhen.

Figur 1 zeigt schematisch Flüssigkeitssysteme einer Dialysemaschine bei einer Hämodialyse. Anstelle oder zusätzlich zu einer Hämodialyse kann eine reine Hämofiltration oder eine Hämodiafiltration realisiert sein. Die Dialysemaschine ist mit einem erfindungsgemäßen System zur Erkennung eines Fehlers im extrakorporalen Blutkreislauf ausgestattet. Das erläuterte Ausführungsbeispiel bezieht sich auf eine venöse Nadeldiskonnektion als eine Art eines Fehlers im extrakorporalen Blutkreislauf.

Generell dargestellt in Figur 1 ist ein extrakorporales System in Form eines extrakorporalen Blutkreislaufs (ECB) sowie ein Dialysierflüssigkeitssystem in Form eines Dialysierflüssigkeitskreislaufs (DKL).

Der extrakorporale Blutkreislauf (ECB) verbindet den Dialysepatienten 12 mit der Dialysemaschine. Während einer Therapie wird dem Patienten 12 Blut über eine arterielle Kanüle A entnommen und mittels einer in einer arteriellen Blutleitung angeordneten arteriellen Blutpumpe 1 über eine arterielle Luftfalle 2 zu einem Dialysator 3 gefördert. In dem Dialysator 3 findet die eigentliche Behandlung, hier Reinigung, des Bluts statt. Vom Dialysator 3 strömt das Blut über eine venöse Luftfalle 7 in einer venösen Blutleitung und über eine venöse Kanüle V zurück in den Patienten 12.

Der Dialysierflüssigkeitskreislauf weist eine Dialysierflüssigkeitspumpe 9, eine Bilanzierungseinrichtung 10 und eine Ultrafiltrationspumpe 11 auf. Mittels der Dialysierflüssigkeitspumpe 9 wird Dialysierflüssigkeit im Gegenstrom zum Blut durch den Dialysator 3 gefördert. Die Bilanzierungseinrichtung 10 dient einer Bilanzierung der Dialysierflüssigkeit des Dialysierflüssigkeitskreislaufs, damit dem Patienten 12 nicht unkontrolliert Wasser entzogen (Dehydrieren) bzw. ihm zu viel Wasser zugeführt wird (Überwässern).

Der Dialysator 3 besteht in der Regel im Wesentlichen aus zahlreichen Hohlfasern mit jeweils einer semipermeablen Membran. In dem anhand der Figur 1 erläuterten Beispiel (Hämodialyse) befindet sich Blut auf einer Seite der Membran, nämlich auf der Seite des extrakorporalen Blutkreislaufs (ECB), und eine Elektrolytlösung, die sogenannte Dialysierflüssigkeit, auf der anderen Seite, nämlich auf der Seite des Dialysierflüssigkeitskreislaufes (DKL). Über die Membran des Dialysators 3 findet Diffusion und Konvektion statt, so dass das Blut gereinigt wird.

Das Ausführungsbeispiel gemäß Figur 1 kann weiterhin nicht dargestellte zusätzliche Messeinrichtungen, Pumpen, Blasenfänger usw. umfassen.

Zur Überwachung der Therapievorgänge dienen Druckaufnehmer oder Drucksensoren 4, 5, 6. Im Einzelnen sind dies ein Eingangsdruckaufnehmer 4 im Bereich der Luftfalle 2, ein arterieller Druckaufnehmer 5 zwischen der arteriellen Kanüle A und der arteriellen Blutpumpe 1 sowie ein venöser Druckaufnehmer 6 im Bereich der venösen Luftfalle 7. Im Folgenden wird exemplarisch der venöse Drucksensor 6 detailliert betrachtet.

Der venöse Druckaufnehmer 6 misst den Druck PV zwischen Luftfalle 7 bzw. Dialysator 3 und dem venösen Zugang V des Patienten. PV setzt sich im Normalfall aus dem durch die Blutpumpe 1 erzeugten Druck und dem Druck im venösen Zugang V des Patienten zusammen. Liegt ein Fehler im extrakorporalen Blutkreislauf in Form einer venösen Nadeldiskonnektion (VND) vor, kommt es zu einem Druckabfall beim venösen Zugang V, wodurch mittels des Druckaufnehmers 6 ein Druckabfall in PV (venöser Druck) festgestellt wird. Durch den mit einer VND korrespondierenden Druckabfall ist PV ein möglicher Sensorwert, der genutzt werden kann, um im Rahmen der vorliegenden Erfindung einen FEB zu detektieren.

Zur Überwachung der Dialysemaschine kommen nach der Erfindung zumindest drei Komponenten zum Einsatz: wenigstens zwei Bewertungseinheiten, die jeweils ein Bewertungskriterium zur Erkennung eines FEB bestimmen, wenigstens zwei Überwachungseinheiten, die aus den bestimmten Bewertungskriterien der Bewertungseinheit jeweils einen FEB ermitteln, und eine Kombinierungseinheit, mit der nach der Erfindung die Überwachungseinheiten und damit auch die Bewertungseinheiten kombiniert werden. Eine, mehrere oder alle genannten drei Einheiten können jeweils eine Speichereinheit, eine Recheneinheit, eine Energieversorgung und eine Datenleitung aufweisen.

Eine Bewertungseinheit bestimmt für einen bestimmten, mittels eines geeigneten Sensors vor, während oder nach einer Therapie erfassten Zustandsparameter ein Bewertungskriterium zur Erkennung eines FEB. Beispiele für Bewertungseinheiten werden später erläutert. Die Bewertungseinheiten sind zur Bewertung der Sensorwerte im extrakorporalen Blutkreislauf beliebig wählbar und hängen von der Art des Sensorwerts ab. Die Bewertungseinheiten können sowohl als eigenständige Einheiten als auch als logische Einheiten mit der jeweiligen Überwachungseinheit ausgebildet sein.

Figur 2 zeigt eine schematische Darstellung eines Zustandsautomaten für ein erfindungsgemäßes System. Eine Übersicht über die einzelnen Komponenten und ihre Anbindung an die Dialysemaschine verschafft Figur 3.

Der in Figur 2 dargestellte Zustandsautomat für ein erfindungsgemäßes System zeigt beispielhaft Zustände auf, die zur singulären/noch nicht kombinierten Erkennung eines FEB's mittels eines erfassten Zustandsparameters (Sensorwert) und eines darauf angewendeten Bewertungskriteriums durchlaufen werden. In einem Initialisierungszustand werden mittels einer beliebigen Bewertungseinheit bewertete Zustandsparameter initialisiert. Die Bewertungseinheit kann zum Beispiel die Grenzen eines venösen Drucks PV als Bewertungskriterien bestimmen.

Nach einer Ausführungsform kann das System in dem Initialisierungszustand verbleiben bzw. der Initialisierungszustand so lange beibehalten werden, bis ein gewünschter Startzeitpunkt der Bewertungseinheit erreicht ist. Die Initialisierung erfolgt in vorteilhafter Weise zu Beginn einer Therapie. Eine Reinitialisierung wiederum erfolgt vorteilhafter Weise nachdem eine Störgröße erkannt wurde und/oder nachdem ein Alarm ausgelöst wurde. Dabei können Störgrößen Faktoren sein, die ein Bewertungskriterium negativ beeinflussen können, so dass ein FEB nicht erkannt wird oder in falscher Weise (Fehlalarm) erkannt wird.

Nachdem die Startzeit erreicht ist, wechselt die Bewertungseinheit bei einer Ausführungsform der Erfindung in einen Bewertungszustand. In diesem wird der Bewertungseinheit ein Sensorwert als Eingabe sowie ggf. zusätzlich eine mögliche Störgröße mit beliebiger Abtastrate zugeführt. Bei einer Veränderung der Störgröße können das entsprechende Bewertungskriterium und die Bewertungseinheit reinitialisiert werden. Die Reinitialisierung kann erreicht werden, indem wieder in den Initialisierungszustand gewechselt wird.

Im Bewertungszustand wird vorzugsweise fortwährend ein Bewertungskriterium für die mittels des Sensors erfassten Zustandsparameter definiert. Dieses wird genutzt, um einen FEB zu erkennen. Zum Beispiel wird im Detektierungszustand bei Zustandsparametern (z.B. PV-Werte) unterhalb einer als Bewertungskriterium definierten Grenze ein FEB vermutet.

Nach einer Option der Erfindung kann eine Zeitdauer, im Folgenden als Erkennungszeitraum ZEIT bezeichnet, zur Erkennung eines FEB definiert werden. Liegt der sensorgemessene Zustandsparameter länger als ein ausgewählter Erkennungszeitraum außerhalb definierter Bewertungskriterien (Grenzen), wird auf ein Vorliegen eines FEB geschlossen und ein solcher erkannt.

Ein Erkennungszeitraum ZEIT, der für eine Erkennung eines FEB überschritten werden muss, kann in vorteilhafter Weise genutzt werden, um Fehlalarme, zum Beispiel verursacht durch kurzfristige Zustandsparameterabweichungen, z.B. Druckschwankungen, zu vermeiden. Typischerweise wird der Erkennungszeitraum ZEIT in Abhängigkeit der Charakteristika einer Bewertungseinheit bestimmt und nicht anhand von Veränderungen in Sensorwerten gesetzt, wie es z.B. aus dem Patent EP 1 815 878 B1 bekannt ist. Wenn ein Sensorwert nicht länger als der Erkennungszeitraum ZEIT jenseits der Bewertungsgrenzen liegt, kann ein entsprechender Zähler in der Recheneinheit der betreffenden Überwachungseinheit zurückgesetzt werden, so dass die Bewertungseinheit anhand der Sensorwerte ein neues Bewertungskriterium berechnet. Wird jedoch der Erkennungszeitraum ZEIT bei einem Bewertungskriterium überschritten, wird in einen Alarmierungszustand gewechselt. In diesem wird ein Alarm ausgelöst. Mögliche Folgen eines Alarms sind vorzugsweise ein Anhalten der Blutpumpe (Aktor), ein Schließen der venösen Schlauchabsperrklemme sowie eine Alarmierung des Patienten und/oder von Pflegepersonal durch akustische und/oder visuelle Signale oder Ähnliches. Nach einer entsprechenden Überprüfung und Quittierung wird vom Alarmierungszustand zurück in den Initialisierungszustand gewechselt. In diesem beginnt der zuvor beschriebene Ablauf zur Erkennung eines FEB von vorne.

Mit dem extrakorporalen Blutkreislauf ECB der in Figur 1 dargestellten Dialysemaschine sind Sensoreinheiten wirkverbunden, z.B. die Drucksensoren 4, 5, 6. Figur 3 zeigt die Verwendung von n Sensoreinheiten, beispielhaft eine erste Sensoreinheit 1 und eine n-te Sensoreinheit n, wobei n eine ganze natürliche Zahl ist. Im Falle von z.B. drei Sensoreinheiten ist n gleich 3, im Falle von fünf Sensoreinheiten ist n gleich 5, usw.

Jede Sensoreinheit ist mit einer Bewertungseinheit 18, 19, 20 verbunden und leitet erfasste Zustandsparameter/Sensorwerte an diese weiter. Im Beispiel der Figur 3 ist die erste Sensoreinheit, z.B. der Druckaufnehmer 4, mit einer ersten Bewertungseinheit 18 und nachfolgend einer ersten Überwachungseinheit 13, die zweite Sensoreinheit, z. B. der Druckaufnehmer 5 mit einer zweiten Bewertungseinheit 19 und nachfolgend einer zweiten Überwachungseinheit 14 und die n-te Sensoreinheit, z.B. der Druckaufnehmer 6 mit einer n-ten Bewertungseinheit 20 und nachfolgend einer n-ten Überwachungseinheit 15 verbunden. Jede Bewertungseinheit 18, 19, 20 und jede Überwachungseinheit 13, 14, 15 weist jeweils eine Speichereinheit und eine Recheneinheit auf.

Die Überwachungseinheiten 1 bis n (13, 14, 15) sind schließlich mit einer Kombinierungseinheit 16 verbunden. Dort werden die von den Überwachungseinheiten 13, 14, 15 übermittelten Signale verarbeitet (kombiniert) und zu einem kombinierten Signal verknüpft. Der Ausgang der Kombinierungseinheit 16 wird zur Steuerung oder Regelung der Dialysemaschine genutzt, was durch eine Signalleitung 17 angedeutet ist.

Nach der Darstellung der Figur 3 ist jeweils einer Sensoreinheit 4, 5, 6 eine Bewertungseinheit 18, 19, 20 zugewiesen.

Die Ergebnisse der einzelnen Überwachungseinheiten werden in der Kombinierungseinheit zusammengefasst, nachdem sie via Datenleitungen übertragen wurden. Auch diese Einheit kann zur Kombinierung den entsprechenden zuvor beschriebenen Zustandsautomaten sowie eine Recheneinheit, eine Speichereinheit, Datenleitungen und eine Energieversorgung zur Erkennung einer VND / eines FEB nutzen. In dem hier aufgeführten Beispiel steuert die Kombinierungseinheit 16 entsprechende Aktoren, unter anderem eine venöse Schlauchabsperrklemme 8 und die Blutpumpe 1 im extrakorporalen Blutkreislauf über die Datenleitung 17, so dass ein sicherer Zustand gewährleistet ist, falls ein Fehler im extrakorporalen Blutkreislauf erkannt wird.

Figur 3 veranschaulicht die Kombination mehrerer Bewertungseinheiten 18, 19, 20 und Überwachungseinheiten 13, 14, 15 mit einer Kombinierungseinheit 16. Die Überwachungseinheiten 13, 14, 15 überwachen unabhängig voneinander beliebige Sensorwerte zur jeweiligen singulären Erkennung eines FEB's. Dabei können beliebige, unterschiedliche oder gleiche Bewertungseinheiten 18, 19, 20 und auch gleiche oder verschiedene Sensorwerte genutzt werden.

Entsprechend Fig. 4 können einer Sensoreinheit 4 auch mehrere Bewertungseinheiten 18, 19 und/oder Überwachungseinheiten 13, 14 zugewiesen sein. Das heißt, dass mittels einer Sensoreinheit erfasste Zustandsparameter (Sensorwerte) nach der Erfindung nicht nur durch eine Bewertungseinheit und der dieser zugewiesenen Überwachungseinheit verarbeitet werden können, sondern die Zustandsparameter (Sensorwerte) auch mehreren Bewertungseinheiten zugeführt werden können, wo sie je nach Bewertungseinheit individuell verarbeitet und an die entsprechende Überwachungseinheit geleitet werden.

Zu jedem Zeitpunkt der Therapie hat jede Überwachungseinheit eine Variable isFEB als Ausgang der Erkennung von Fehlern im extrakorporalen Blutkreislauf. Die Variablen isFEB sind in der Figur 3 der jeweiligen Überwachungseinheit entsprechend nummeriert und heißen dort $isFEB_1$ (für die erste Überwachungseinheit 13), $isFEB_2$ (für die zweite Überwachungseinheit 14) und $isFEB_n$ (für die n-te Überwachungseinheit 15). Die Variable isFEB ist 1 im Falle eines erkannten Fehlers und 0 für den Fall, wenn kein FEB erkannt wurde. Dabei sei aber darauf hingewiesen, dass die Variable auch einen Wert zwischen 0 und 1 annehmen kann. In der Kombinierungseinheit 16 findet eine Kombinierung der einzelnen Überwachungseinheiten - die verschiedene Bewertungseinheiten zur Erkennung eines FEB nutzen - statt. Die Variable isFEB einer jeden Überwachungseinheit kann mit einem Gewicht $\omega$, welches für jede Überwachungseinheit definiert ist, multipliziert werden. Das Produkt einer jeden Multiplikation kann für alle Überwachungseinheiten in der Recheneinheit aufaddiert werden. Anders ausgedrückt kann mit dem Gewicht $\omega$ die gewichtete Summe der Ausgänge isFEB der Überwachungseinheiten gebildet werden. In dem Fall, dass diese Summe einen Grenzwert $\theta$ überschreitet, kann vom Vorliegen eines FEB ausgegangen werden und eine Alarmierung veranlasst werden.

Die zuvor beschriebene Kombination ist nur eine von zahlreichen möglichen Varianten. Es liegt im Bereich der Erfindung, die Überwachungseinheiten mit allen sinnvollen mathematischen Methoden und Modellen zu kombinieren. Mögliche andere Kombinationsvarianten stammen beispielsweise aus dem Bereich des maschinellen Lernens, wie zum Beispiel gewichtete oder ungewichtete Fallanalysen, Fuzzy-Modelle, neuronale Netze, SVR's oder physikalische oder mathematische Modelle, die z.B. von der Temperatur oder von anderen physikalischen Größen abhängig sind.
Durch das Vorsehen mehrerer einzelner Überwachungseinheiten und deren Kombination mittels der Kombinierungseinheit wird ermöglicht, dass einzelne Bewertungseinheiten einen beliebig gewichteten Anteil an der Erkennung eines FEB haben können. Dies wird durch eine geeignete Wahl des Gewichts $\omega$ und des Grenzwerts $\theta$ erreicht. Des Weiteren kann durch eine geeignete Auswahl der jeweiligen Startzeit der einzelnen Bewertungseinheiten eine Startreihenfolge festgelegt werden, in der die nachfolgenden Überwachungseinheiten eine Überwachung von Zustandsparametern beginnen. Darüber hinaus kann mit einer geeigneten Startzeit außerdem eine zeitliche Verzögerung der Startzeitpunkte der Bewertungseinheiten festgelegt werden. Diese Eigenschaften ermöglichen, dass die Bewertungseinheiten ihre Bewertungskriterien (Initial) anhand von Sensorwerten zu verschiedenen Zeitpunkten berechnen und somit ein gestaffeltes Alarmsystem darstellen. Dies kann mit Vorteil die Anzahl oder Häufigkeit von Fehlalarmen verringern oder sogar minimieren.

Im Beispiel der Figuren 5 und 6 berechnet eine erste Bewertungseinheit ein erstes Bewertungskriterium zur Erkennung eines FEB anhand des mittels des venösen Druckaufnehmers 6 erfassten venösen Drucks PV als erster Zustandsparameter. Dieser Vorgang ergibt sich unter anderem aus Figur 5, in der auf der Abszisse die Zeit t in Sekunden und auf der Ordinate der venöse Druck PV in mmHG aufgetragen ist. Mittels einer durchgezogenen Linie ist der Verlauf des venösen Drucks PV dargestellt. Mittels der ersten Bewertungseinheit werden Bewertungskriterien in Form einer Obergrenze (UCL, gestrichelte Linie) und einer Untergrenze (LCL, strichpunktierte Linie) für den venösen Druck berechnet. Auf Figur 5 und die Art der Berechnung wird später noch detailliert eingegangen.

Eine zweite Bewertungseinheit berechnet ein zweites Bewertungskriterium anhand eines Zustandparameters, im vorliegenden Beispiel wiederum anhand des venösen Drucks PV aufgenommen mittels des venösen Druckaufnehmers 6. Dieser Vorgang ergibt sich unter anderem aus Figur 6, in der auf der Abszisse die Zeit t in Sekunden und auf der Ordinate der venöse Druck PV in mmHG aufgetragen ist. Mittels einer durchgezogenen Linie ist der Verlauf des venösen Drucks PV dargestellt. Mittels der zweiten Bewertungseinheit werden eine (zweite) Obergrenze (UCL, gestrichelte Linie) und eine (zweite) Untergrenze (LCL, strichpunktierte Linie) für den venösen Druck berechnet. Auf Figur 6 und die Art der Berechnung wird später noch detailliert eingegangen.

Es können weitere Bewertungseinheiten genutzt werden, die für gleiche oder andere Zustandsparameter mittels identischer, ähnlicher oder anderer Berechnungsverfahren weitere Bewertungskriterien für das Vorliegen eines FEB berechnen. Diesbezüglich wird auf die Darstellung der Figur 3 verwiesen.

Nach der Erfindung wird mittels einer Überwachungseinheit anhand eines mittels einer Bewertungseinheit berechneten Bewertungskriteriums ein FEB ermittelt. Bezogen auf das Beispiel der Figuren 5 und 6 bedeutet das, dass eine erste Überwachungseinheit, z.B. die Überwachungseinheit 13, anhand des mittels der ersten Bewertungseinheit 18 berechneten Bewertungskriteriums (PV liegt zwischen der ersten Untergrenze und der ersten Obergrenze) das Vorliegen eines FEB ermittelt. Zum Beispiel erkennt die erste Überwachungseinheit PV Werte außerhalb der in Figur 5 dargestellten Grenze, die über einen längeren Zeitraum vorliegen, als venöse Nadeldiskonnektion. Eine zweite Überwachungseinheit, z.B. die Überwachungseinheit 14, ermittelt anhand des mittels der zweiten Bewertungseinheit 19 berechneten Bewertungskriteriums (PV liegt zwischen der zweiten Untergrenze und der zweiten Obergrenze) das Vorliegen eines FEB. Zum Beispiel erkennt die zweite Überwachungseinheit PV Werte außerhalb der in Figur 6 dargestellten Grenze, die über einen längeren Zeitraum vorliegen, als venöse Nadeldiskonnektion. Eine weitere Überwachungseinheit oder weitere Überwachungseinheiten können anhand von mittels einer weiteren Bewertungseinheit oder mehrerer weiterer Bewertungseinheiten berechneten Bewertungskriterien das Vorliegen eines FEB ermitteln.

Jede Überwachungseinheit generiert ein Fehlersignal, das der Kombinierungseinheit 16 zugeführt wird. Diese wird genutzt, um beliebig viele Überwachungseinheiten und damit auch die von diesen ausgegebene Fehlersignale zu kombinieren. Diese Kombination ist von entscheidendem Vorteil. Es wird nicht nur anhand eines bewerteten Fehlersignals auf das Vorliegen eines FEB geschlossen, wie aus dem Stand der Technik bekannt, sondern es wird eine Mehrzahl vorzugsweise unterschiedlich bewerteter Fehlersignale in der Kombinierungseinheit verarbeitet. Die Stärken und Schwächen der jeweiligen Bewertungseinheiten und der darin durchgeführten Bewertungen sind bekannt, und so kann durch gezielte Auswahl oder Verarbeitung der an die Kombinierungseinheit geleiteten Fehlersignale die Qualität der Fehlererkennung und -anzeige verbessert und Fehlalarme können minimiert werden.

In dem Beispiel der Figuren 5 und 6 ist der mittels des venösen Druckaufnehmers 6 erfasste venöse Druck PV der Zustandsparameter, der bewertet wird. In der ersten Bewertungseinheit 18 werden die vorstehend genannten Bewertungskriterien in Form der Obergrenze (UCL) und der Untergrenze (LCL) des venösen Drucks anhand polynomialer Regression bestimmt, wie nachfolgend näher erläutert wird.

Die Gleichung für die verwendete Polynomiale Regression lautet:

$$y(t) = w_0 + w_1 \cdot t + w_2 \cdot t^2 + ... + w_M \cdot t^M = \sum_{j=0}^{M} w_j \cdot t^j$$

In dieser Gleichung ist w das Gewicht eines Monoms, t der Index der Therapiezeit und M ist die höchste Ordnung des Polynoms. Durch eine Berechnung gemäß dieser Gleichung wird der erfasste Zustandsparameter (PV-Wert des venösen Druckaufnehmers 6 als Sensor) modelliert. Eine dabei auftretende Abweichung, der sogenannte Approximations-Fehler, zwischen dem Modell des Sensorwerts und den realen Sensorwerten wird genutzt, um mit Hilfe der nachfolgenden Gleichungen

$$UCL_t := y(t) + k \cdot \sqrt{\frac{\sigma^2}{t}}$$

$$CenterPoint_t := y(t)$$

$$LCL_t := y(t) - k \cdot \sqrt{\frac{\sigma^2}{t}}$$

die Bewertungskriterien in Form der Untergrenze (LCL für lower control limit) sowie der Obergrenze (UCL für upper control limit) zu bestimmen. Dabei ist k ein Faktor, welcher die Weite des Grenzwertfensters bestimmt. Der Index der Therapizeit t wird genutzt um den gemittelten Approximationsfehler von $\sigma^2$ zu einem bestimmten Zeitpunkt der Therapie zu ermitteln. Von diesem gemittelten Fehler wird dann die Wurzel gezogen, um die Abweichung zu einem beliebigen Index t zu bestimmen. Diese Abweichung multipliziert mit dem Faktor k plus bzw. minus den modellierten Sensorwert ergibt die obere Grenze des venösen Drucks (UCL = upper control limit) bzw. die untere Grenze des venösen Drucks (LCL = lower control limit).

In Figur 5 sind die Bewertungskriterien, die mittels der zuvor beschriebenen Bewertungseinheit in Form einer polynomialen Regression bestimmt wurden, dargestellt. Anhand des erfassten venösen Drucks PV und mittels polynomialer Regression werden die Bewertungskriterien in Form der Grenzen LCL und UCL über 140 Sekunden berechnet. Nach etwa 125 Sekunden, der Zeitpunkt ist mit DC (für disconnected) gekennzeichnet, erfolgt im dargestellten Beispiel eine venöse Nadeldiskonnektion (VND).

In Figur 5 stellt die "Centre Line" CL eine Modellierung des venösen Drucks PV mit einer Polynomialen Regression der Ordnung eins dar. Für die Berechnung der Grenzen UCL und LCL wurde ein Wert von k gleich vier gewählt. Zu Beginn der Berechnung wurden die Werte in die Nähe der Pulsation in PV gesetzt. Zum Zeitpunkt DC der venösen Nadeldiskonnektion öffnen sich die Grenzen. Die Variable ZEIT in der Überwachungseinheit muss derart ausgewählt werden, dass die VND rechtzeitig erkannt wird, insbesondere bevor die Grenzen zu weit geöffnet sind. In diesem Beispiel ist nur die Untergrenze LCL zur Erkennung der VND notwendig, da die Grenze von einer entsprechenden Überwachungseinheit genutzt wird, um bei PV Werten unterhalb von LCL eine VND zu erkennen. Bei anderen Sensorwerten oder anderen Fehlern im extrakorporalen Blutkreislauf kann diese Obergrenze UCL aber ebenfalls eine Rolle zur Erkennung spielen.

Einige Vor- und Nachteile der Polynomialen Regression ergeben sich aus Figur 5. Nach einem Zeitablauf von etwa 75 Sekunden ist ein kurzzeitiges Ansteigen des venösen Drucks in Form einer Pulsspitze zu erkennen. Hierdurch kommt es unmittelbar oder sehr schnell zu einem Aufweiten von Ober- und Untergrenze. Eine solche schnelle Reaktion auf eine kurzeitige unproblematische Pulsspitze ist wegen des Aufweitens der Bewertungskriterien von Nachteil. Das Phänomen ist auch nach dem Auftreten der Diskonnektion DC zu erkennen. Es ist jedoch von Vorteil, dass nach Beginn der Messung die Bewertungskriterien in Form von Ober- und Untergrenze sehr schnell, nach ca. 2 bis 3 Sekunden, bestimmt sind und eine Überwachung hinsichtlich von FEB möglich ist.

In der zweiten Bewertungseinheit (siehe Figur 6) werden die Bewertungskriterien in Form der Obergrenze (UCL) und der Untergrenze (LCL) des venösen Drucks anhand eines "gemittelten exponentiellen gewichteten Mittels" bestimmt, wie nachfolgend näher erläutert wird.

Im Wesentlichen beruht die zweite Bewertungseinheit auf einem exponentiell gewichteten Mittel und einer "heuristischen" Varianz zur Bestimmung der Bewertungskriterien in Form der Grenzen UCL und LCL. Im Folgenden wird diese Bewertungseinheit auch als EWMA bezeichnet. Es wird zunächst an der Gleichung

$$\lambda_t = (1-\tilde{\lambda}) \cdot \lambda_{t-1} + \tilde{\lambda} \cdot \lambda_\infty$$

das Gewicht $\lambda_t$ bestimmt, mit welchem ein Sensorwert, hier der venöse Druck, in das Mittel eingeht. Dies ist eine rekursive Formel und der Parameter $\tilde{\lambda}$ bestimmt die Gewichtsabnahme pro rekursivem Schritt. $\lambda_\infty$ bestimmt einen asymptotischen Wert des Gewichts, gegen den das Gewicht $\lambda_t$ konvergiert. Die Gleichung

$$Z_t := (1-\lambda_t) \cdot Z_{t-1} + \lambda_t \cdot X_t$$

gibt das eigentliche exponentiell gewichtete Mittel an. Diese Gleichung ist eine rekursive Gleichung und nutzt neben dem Gewicht auch den Sensorwert X, um einen Mittelwert zu berechnen. Das Ergebnis einer Berechnung mittels dieser Gleichung wird unter Verwendung der folgenden Berechnung

$$p_t := \frac{1}{t} \cdot \sum_{i=1}^{t} Z_i, \textit{ für } t \geq 1$$

nochmals gemittelt. Auf diese Weise wird in vorteilhafter Weise erreicht, dass die Bestimmung robuster gegenüber kurzfristigen Schwankungen des Sensorwerts ist. Das gemittelte Ergebnis $p_t$ wird in der Berechnung der Varianz nach der folgenden Gleichung

$$V[p_t] = \frac{p_t(1-p_t)}{t} \cdot \left(1 - \frac{1}{t} \cdot \frac{1-\lambda_t}{\lambda_t(2-\lambda_t)} \cdot \left(1-(1-\lambda_t)^t\right) \cdot \left(2 + (1-\lambda_t) \cdot \left(1-(1-\lambda_t)^t\right)\right)\right)$$

mit dem aktuellen Gewicht $\lambda_t$ genutzt, um die Varianz $V[p_t]$ zu berechnen. Das Ergebnis der Varianz $V[p_t]$ definiert zusammen mit dem Faktor k und dem Wert $p$ zu jedem Zeitpunkt die Bewertungskriterien in Form der Obergrenze UCL bzw. der Untergrenze LCL gemäß den nachfolgenden Gleichungen:

$$UCL_t := p_t + k \cdot \sqrt{V[p_t]}$$

$CenterPoin't_t := p_t$

$$LCL_t := p_t - k \cdot \sqrt{V[p_t]}$$

In Figur 6 sind die mittels der vorgenannten Berechnung bestimmten Bewertungskriterien dargestellt. Für einen Zeitraum von 650 Sekunden wurden Sensorwerte PV des venösen Druckaufnehmers als Zustandsparameter erfasst. Nach einer Dauer von ca. 570 Sekunden kommt es zu einer venösen Nadeldiskonnektion (VND), welcher Zeitpunkt als DC (disconnected) gekennzeichnet ist.

Die "Centre Line" wurde durch $p$ berechnet. Des Weiteren wurden ein Faktor von $k$ = 3, $\lambda_\infty$ = 0,0095 und $\tilde{\lambda}$ = 0,01 zur Berechnung genutzt. Der erste Wert des Gewichts $\lambda_t$ wurde mit 1 initialisiert.

In Figur 6 ist dargestellt, dass sich die gemäß der vorstehenden Berechnung bestimmten Bewertungskriterien in Form der Obergrenze UCL und Untergrenze LCL an PV annähern. Allerdings benötigt dieser Vorgang eine gewisse Zeit, was bei einer Nutzung der zweiten Bewertungseinheit zur Erkennung eines FEB kurz nach Beginn der Überwachung, vorliegend etwa innerhalb der ersten ca. 300 Sekunden, von Nachteil ist. Nachdem jedoch die Bewertungskriterien in Form der Obergrenze UCL und Untergrenze LCL nach Ablauf dieser Zeit genügend nahe an der Pulsation PV sind, sind sie robust gegenüber Veränderungen, wie zum Zeitpunkt DC der VND zu erkennen ist. Auch hier ist nur LCL notwendig zur Erkennung einer VND, jedoch kann auch UCL bei anderen Sensorwerten oder FEB's relevant sein.

Sollte nun ein FEB anhand einer oder beider der vorgenannten Bewertungskriterien festgestellt und ein Fehlersignal ausgegeben werden, erfolgt nach der Erfindung eine Kombination in einer Kombinierungseinheit. Im vorliegenden Beispiel wurde zum Bestimmen der optimalen Parameter für die Kombinierungseinheit ein Grid-Search Algorithmus genutzt. Dieser ist ein Optimierungsverfahren, das auf einem Testdatensatz von Therapien mit FEB und ohne FEB angewendet wurde. Auf diese Weise war es möglich, möglichst gut geeignete Parameter bzw. möglichst gute Kombinationen von Überwachungseinheiten zu bestimmen. Dabei werden mittels Grid-Search verschiedene Parameter und Kombinationen getestet. Solche Parameter und Kombinationen mit den wenigsten Fehlalarmen und meisten erkannten FEB's werden als möglichst gute Kombination bzw. möglichst gute Parameter zur Erkennung eines FEB angenommen. Für die Optimierung mittels Grid-Search wurden beispielhaft fünf Therapien mit einer Dauer von jeweils ca. vier Stunden genutzt. Dabei wurden jeweils zehn venöse Nadeldiskonnektionen simuliert. Während jeder der Therapien wurden alle 15 Minuten Maschinenparameter geändert, um so möglichst schwierige Therapiesituationen nachzustellen, die in der Regel oder häufig Fehlalarme auslösen. Der venöse Druckverlauf während der simulierten VNDs wurde unter realistischen Bedingungen, Shuntinnendruck und Shuntfluss wie beim Menschen, aufgenommen, um realistische Daten zu gewährleisten.

Bei der vorstehenden Optimierung anhand der oben beschrieben Datensätze wurden drei Überwachungseinheiten als beste mögliche Kombination durch Grid-Search ermittelt. Dabei wird zweimal eine Polynomiale Regression und einmal eine EWMA als Bewertungskriterium genutzt. Die Überwachungseinheiten werden dabei mit besonderem Vorteil in folgender Reihenfolge mit einer zeitlichen Verschiebung von ca. 60 Sekunden gestartet:

1. Polynomiale Regression
2. Polynomiale Regression
3. EWMA

Dabei waren für jede Überwachungseinheit die Werte für die Gewichte $\omega = 1{,}5$ und für die Grenze zur Erkennung einer FEB $\theta = 1{,}5$. Jede Überwachungseinheit konnte unabhängig von den anderen Überwachungseinheiten eine VND erkennen. Bei der Bestimmung mittels polynomialer Regression mussten die Werte des venösen Drucks PV zur Erkennung einer VND für eine Dauer von etwa zwölf Sekunden (ZEIT) unterhalb der Untergrenze LCL liegen. Bei dem EWMA Verfahren mussten die Werte des venösen Drucks PV für eine Dauer von ca. 60 Sekunden unterhalb der Untergrenze LCL liegen. Die anderen Parameter waren wie zuvor beschrieben und sind in der folgenden Tabelle zusammengefasst:

| | M | K | Zeit[s] | $\lambda_\infty$ | $\tilde{\lambda}$ | Position | w | $\theta$ | Lag[s] |
|---|---|---|---|---|---|---|---|---|---|
| Polynomiale Regression | 1 | 4 | 12 | NA | NA | 1 | 1,5 | NA | NA |
| Polynomiale Regression | 1 | 4 | 12 | NA | NA | 2 | 1,5 | NA | NA |
| EWMA | NA | 3 | 60 | 0,095 | 0,01 | 3 | 1,5 | NA | NA |

Evaluiert wurde diese Kombination mit den genannten Parametern auf 58 VNDs, als Beispiel eines FEB's und anhand von fünfzehn Therapien ohne FEB mit einer jeweiligen Dauer von vier Stunden. Die Datensätze wurden genauso erzeugt wie die Datensätze, welche bei dem vorbeschriebenen Grid-Search-Verfahren genutzt wurden, so dass realistische Bedingungen gewährleistet waren. Durch die Kombinierungseinheit mit der oben beschriebenen Kombination und den oben beschriebenen Parametern wurden 55 von insgesamt 58 VNDs erkannt und 71 Fehlalarme ausgelöst. Im Vergleich dazu hat ein herkömmliches Alarmsystem bei identischen Bedingung nur eine einzige VND erkannt und 77 Fehlalarme ausgelöst. Dies zeigt das Potential der hier vorgestellten Erfindung.

## Patentansprüche

**1.** System zur Erkennung eines Fehlers in einem extrakorporalen Blutkreislauf einer extrakorporalen Blutbehandlungsmaschine, mit

zumindest einem Sensor (4, 5, 6) zur Erfassung zumindest eines den extrakorporalen Blutkreislauf charakterisierenden Zustandsparameters,
zumindest einer Bewertungseinheit (18, 19, 20) zur Bestimmung eines ersten Zustandsparameter-Bewertungskriteriums für ein Erkennen zumindest eines Fehlers in dem extrakorporalen Blutkreislauf,
zumindest einer Überwachungseinheit (13, 14, 15) zum Treffen einer Entscheidung hinsichtlich des Vorliegens zumindest eines Fehlers in dem extrakorporalen Blutkreislauf und zum Generieren eines ersten Fehlersignals sowie zur Überwachung des erfassten Zustandsparameters, jeweils unter Verwendung des ersten Zustandsparameter-Bewertungskriteriums,

wobei

das System zumindest eine weitere Bewertungseinheit aufweist, um zumindest ein weiteres Zustandsparameter-Bewertungskriterium zu bestimmen,
das System zumindest eine weitere Überwachungseinheit (13, 14, 15) aufweist, um unter Verwendung des zumindest einen weiteren Zustandsparameter-Bewertungskriteriums eine Entscheidung hinsichtlich des Vorliegens eines Fehlers in dem extrakorporalen Blutkreislauf zu treffen und zumindest ein weiteres Fehlersignal zu generieren,
das System eine Kombinierungseinheit (16) aufweist, um das erste Fehlersignal und das zumindest eine weitere Fehlersignal zu einem kombinierten Fehlersignal zu kombinieren, und

**dadurch gekennzeichnet, dass**

das System dazu ausgelegt ist, das erste und das zumindest eine weitere Fehlersignal jeweils einer Gewichtung zu unterziehen,
wobei das erste Fehlersignal und das zumindest eine weitere Fehlersignal kombiniert werden, indem die gewichteten Fehlersignale mathematisch verknüpft werden.

**2.** System nach Anspruch 1, wobei jedem Sensor (4, 5, 6) nur eine einzige Bewertungseinheit (18, 19, 20) zur Bestimmung eines einzigen Zustandsparameter-Bewertungskriteriums und vorzugsweise nur eine einzige Überwachungseinheit (13, 14, 15) zugeordnet ist oder dass einem Sensor (4, 5, 6) eine Mehrzahl von Bewertungseinheiten (18, 19, 20) zur Bestimmung einer Mehrzahl von Zustandsparameter-Bewertungskriterien und vorzugsweise eine Mehrzahl von Überwachungseinheiten (13, 14, 15) zugeordnet sind.

**3.** System nach Anspruch 1 oder 2, wobei es Bestandteil der Blutbehandlungsmaschine, beispielsweise einer Dialysemaschine, ist.

**4.** System nach einem der Ansprüche 1 bis 3, mit einer Anzeige- und/oder Alarmeinrichtung zur Erzeugung einer Anzeige und/oder eines Alarms, wenn das kombinierte Fehlersignal einen vorbestimmten Grenzwert in positiver oder negativer Richtung überschreitet und/oder in einen vorbestimmten Wertebereich fällt und/oder mit einem Notaus-Schalter zum Ausschalten der Blutbehandlungsmaschine wenn das kombinierte Fehlersignal einen vorbestimmten Grenzwert in positiver oder negativer Richtung überschreitet und/oder in einen vorbestimmten Wertebereich fällt.

**5.** System nach einem der vorstehenden Ansprüche, das dazu ausgelegt ist, für zumindest einen erfassten Zustandsparameter eine Initialisierung durchzuführen, indem dem Zustandsparameter ein Initialwert zugewiesen wird und das Bewertungskriterium unter Verwendung des Initialwerts sowie nach der Initialisierung erfassten Zustandsparametern bestimmt wird.

**6.** System nach einem der vorstehenden Ansprüche, das dazu ausgelegt ist, dass eine Überwachung eines erfassten Zustandsparameters und ein Feststellen eines Fehlers mittels des ersten Bewertungskriteriums zeitlich versetzt zu einer Überwachung eines erfassten Zustandsparameters und einem Feststellen eines Fehlers mittels des zumindest einen weiteren Bewertungskriteriums abläuft, insbesondere nach einem Systemstart und/oder einer Initialisierung und/oder einer Reinitialisierung.

**7.** System nach einem der vorstehenden Ansprüche, dass dazu ausgelegt ist, eine Störgröße, insbesondere eine Ultrafiltrationsrate, eine Dialysierflüssigkeitsflussrate, eine Blutflussrate, eine Pegelregulierung oder ein vorangegangener Alarm, zu erfassen und diese für eine Entscheidung hinsichtlich des Vorliegens eines Fehlers zu berücksichtigen.

**8.** System nach Anspruch 7, das dazu ausgelegt ist, nach Erfassung einer Störgröße eine Reinitialisierung durchzuführen.

**9.** System nach einem der vorstehenden Ansprüche, bei dem das erste Bewertungskriterium und das zumindest eine weitere Bewertungskriterium identisch oder verschieden sind.

**10.** System nach einem der vorstehenden Ansprüche, das dazu ausgelegt ist, ein Bewertungskriterium mittels polynominaler Regression oder mittels exponentiell gewichtetem gleitendem Mittel zu bestimmen.

11. System nach einem der vorstehenden Ansprüche, bei dem ein überwachter Zustandsparameter der venöse Blutdruck oder der arterielle Blutdruck ist.

## Claims

1. System for detection of an error in an extracorporeal blood circulation of an extracorporeal blood treatment device, having
at least one sensor (4, 5, 6) for detecting at least one state parameter characterising the extracorporeal blood circulation,
at least one evaluation unit (18, 19, 20) for determining a first state parameter-evaluation criterion for a detection of at least one error in the extracorporeal blood circulation,
at least one monitoring unit (13, 14, 15) for making a decision with regard to the presence of at least one error in the extracorporeal blood circulation and for generating a first error signal and for monitoring the detected state parameter in each case by using the first state parameter-evaluation criterion,
wherein
the system has at least one further evaluation unit, in order to determine at least one further state parameter-evaluation criterion,
the system has at least one further monitoring unit (13, 14, 15), in order to make a decision with regard to the presence of an error in the extracorporeal blood circulation by using the at least one further state parameter-evaluation criterion and to generate at least one further error signal,
the system has a combination unit (16), in order to combine the first error signal and the at least one further error signal into a combined error signal, and
**characterised in that**
the system is designed subject the first and the at least one further error signal in each case to a weighing,
wherein the first error signal and the at least one further error signal are combined, by mathematically linking the weighted error signals.

2. System according to claim 1, wherein only a single evaluation unit (18, 19, 20) for determining a single state parameter-evaluation criterion and preferably only a single monitoring unit (13, 14, 15) is assigned to each sensor (4, 5, 6) or that a plurality of evaluation units (18, 19, 20) for determining a plurality of state parameter-evaluation criteria and preferably a plurality of monitoring units (13, 14, 15) are assigned to a sensor (4, 5, 6).

3. System according to claim 1 or 2, wherein it is a component of the blood treatment machine, for example, a dialysis machine.

4. System according to any one of claims 1 to 3, having a display- and/or alarm device for generating a display and/or an alarm, if the combined error signal exceeds a predetermined limit value in the positive or negative direction and/or falls into a predetermined value range and/or having an emergency off switch for switching off the blood treatment machine if the combined error signal exceeds a predetermined limit value in the positive or negative direction and/or falls into a predetermined value range.

5. System according to any one of the preceding claims, which is designed to perform an initialisation for at least one detected state parameter, by assigning an initial value to the state parameter and by determining the evaluation criterion using the initial value and state parameters detected after the initialisation.

6. System according to any one of the preceding claims, which is designed such that a monitoring of a detected state parameter and a detection of an error by means of the first evaluation criterion takes place offset in time to a monitoring of a detected state parameter and a detection of an error by means of the at least one further evaluation criterion, in particular, after a system start and/or an initialisation and/or a reinitialisation.

7. System according to any one of the preceding claims, which is designed to detect disturbance variables, in particular, an ultrafiltration rate, a dialysis liquid flow rate, a blood flow rate, a level regulation or a previous alarm and take these into account in making a decision with regard to the presence of an error.

8. System according to claim 7, which is designed to perform a reinitialisation after detection of a disturbance variable.

9. System according to any one of the preceding claims, in which the first evaluation criterion and the at least one further evaluation criterion are identical or different.

10. System according to any one of the preceding claims, which is designed to determine an evaluation criterion by means of polynomial regression or by means of exponentially weighted sliding means.

11. System according to any one of the preceding claims, in which a monitored state parameter is the venous blood pressor or the arterial blood pressure.

## Revendications

1. Système de reconnaissance d'une erreur dans une circulation sanguine extracorporelle d'une machine extracorporelle de traitement du sang, comportant
au moins un capteur (4, 5, 6) pour la saisie d'au moins un paramètre d'état caractérisant la circulation sanguine extracorporelle,
au moins une unité d'évaluation (18, 19, 20) pour la détermination d'un premier critère d'évaluation du paramètre d'état pour une identification d'au moins une erreur dans la circulation sanguine extracorporelle,
au moins une unité de surveillance (13, 14, 15) pour la prise d'une décision au regard de la présence d'au moins une erreur dans la circulation sanguine extracorporelle et pour la génération d'un premier signal d'erreur ainsi que pour la surveillance du paramètre d'état saisi, respectivement en utilisant le premier critère d'évaluation du paramètre d'état,
dans lequel
le système présente au moins une autre unité d'évaluation, pour déterminer au moins un autre critère d'évaluation de paramètre d'état,
le système présente au moins une autre unité de surveillance (13, 14, 15) pour prendre une décision en utilisant l'au moins un autre critère d'évaluation de paramètre d'état au regard de la présence d'une erreur dans la circulation sanguine extracorporelle et pour générer au moins un autre signal d'erreur,
le système présente une unité de combinaison (16) pour combiner le premier signal d'erreur et l'au moins un autre signal d'erreur en un signal d'erreur combiné, et
**caractérisé en ce que**
le système est conçu pour soumettre le premier et l'au moins un autre signal d'erreur respectivement à une pondération,
dans lequel le premier signal d'erreur et l'au moins un autre signal d'erreur sont combinés, en reliant mathématiquement les signaux d'erreur pondérés.

2. Système selon la revendication 1, dans lequel à chaque capteur (4, 5, 6) n'est affectée qu'une seule unité d'évaluation (18, 19, 20) pour la détermination d'un seul critère d'évaluation de paramètre d'état et de préférence qu'à une seule unité de surveillance (13, 14, 15) ou en ce qu'à un capteur (4, 5, 6) est affectée une pluralité d'unités d'évaluation (18, 19, 20) pour la détermination d'une pluralité de critères d'évaluation de paramètres d'état et de préférence une pluralité d'unités de surveillance (13, 14, 15).

3. Système selon la revendication 1 ou 2, lequel est un composant de la machine de traitement du sang, par exemple d'une machine de dialyse.

4. Système selon l'une quelconque des revendications 1 à 3, comportant un dispositif de notification et/ou d'alarme pour la production d'une notification et/ou d'une alarme, lorsque le signal d'erreur combiné dépasse une valeur seuil prédéterminée dans un sens positif ou négatif et/ou se situe dans une plage de valeurs prédéterminée et/ou comportant un bouton d'arrêt d'urgence pour déconnecter la machine de traitement du sang lorsque le signal d'erreur combiné dépasse une valeur seuil prédéterminée dans un sens positif ou négatif et/ou se situe dans une plage de valeur prédéterminée.

5. Système selon l'une quelconque des revendications précédentes, qui est conçu pour réaliser une initialisation pour au moins un paramètre d'état saisi, en attribuant au paramètre d'état une valeur initiale et le critère d'évaluation est déterminé en utilisant la valeur initiale ainsi que des paramètres d'états saisis après l'initialisation.

6. Système selon l'une quelconque des revendications précédentes, qui est conçu de telle sorte qu'une surveillance d'un paramètre d'état saisi et une constatation d'une erreur au moyen du premier critère d'évaluation s'effectuent de façon décalée dans le temps en une surveillance d'un paramètre d'état saisi et une constatation d'une erreur au moyen de l'au moins un autre critère d'évaluation, en particulier après un lancement du système et/ou une initialisation et/ou une réinitialisation.

**7.** Système selon l'une quelconque des revendications précédentes, qui est conçu pour saisir une valeur perturbatrice, en particulier un taux d'ultrafiltration, un débit de fluide de dialyse, un débit sanguin, une régulation de niveau ou une alarme précédente et pour prendre en compte ceux-ci pour une décision au regard de la présence d'une erreur.

**8.** Système selon la revendication 7, qui est conçu pour réaliser une réinitialisation après une saisie d'une valeur perturbatrice.

**9.** Système selon l'une quelconque des revendications précédentes, dans lequel le premier critère d'évaluation et l'au moins un autre critère d'évaluation sont identiques ou différents.

**10.** Système selon l'une quelconque des revendications précédentes, qui est conçu pour déterminer un critère d'évaluation au moyen d'une régression polynomiale ou au moyen d'une moyenne mobile pondérée de façon exponentielle.

**11.** Système selon l'une quelconque des revendications précédentes, dans lequel un paramètre d'état surveillé est la pression sanguine veineuse ou la pression sanguine artérielle.

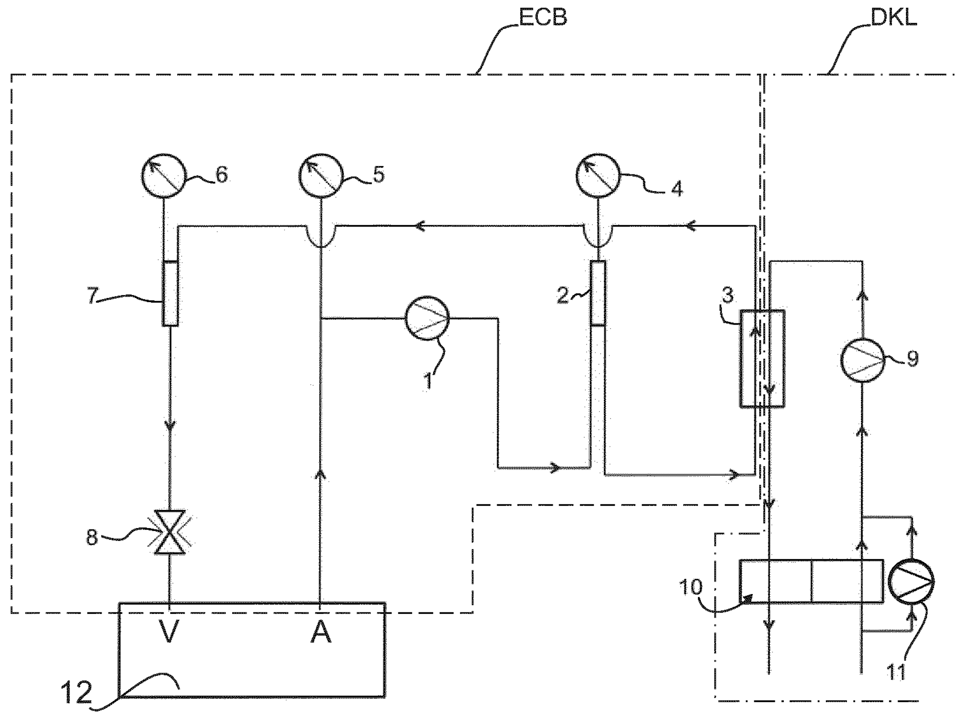
ECB
DKL
6
5
4
7
2
3
1
9
8
10
11
V
A
12

Fig.1

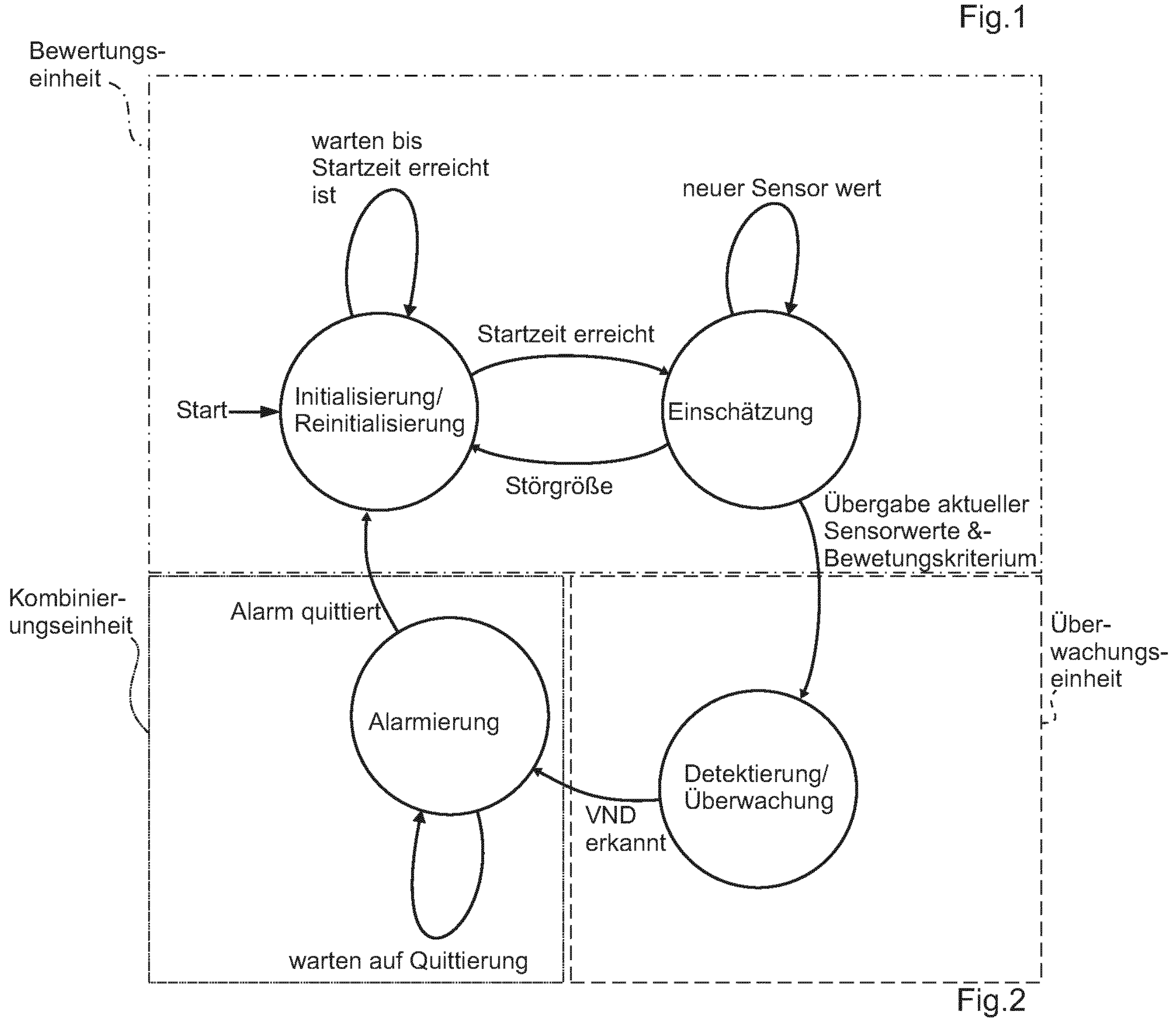
Bewertungs-
einheit
warten bis
Startzeit erreicht
ist
neuer Sensor wert
Startzeit erreicht
Start
Initialisierung/
Reinitialisierung
Einschätzung
Störgröße
Übergabe aktueller
Sensorwerte &-
Bewetungskriterium
Kombinier-
ungseinheit
Alarm quittiert
Über-
wachungs-
einheit
Alarmierung
Detektierung/
Überwachung
VND
erkannt
warten auf Quittierung

Fig.2

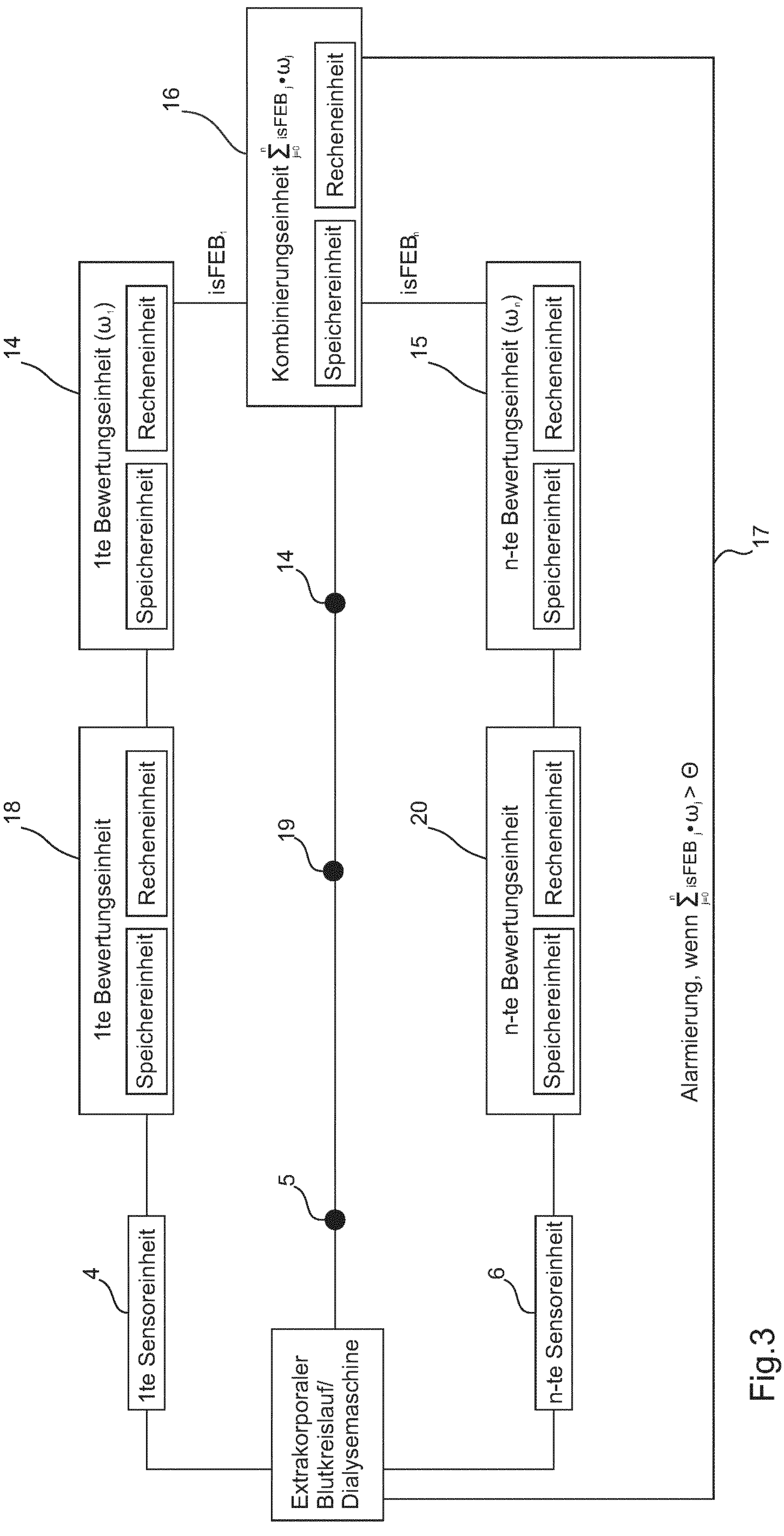
Extrakorporaler Blutkreislauf/ Dialysemaschine
1te Sensoreinheit
4
n-te Sensoreinheit
6
5
19
14
1te Bewertungseinheit
Speichereinheit
Recheneinheit
18
n-te Bewertungseinheit
Speichereinheit
Recheneinheit
20
1te Bewertungseinheit (ω1)
Speichereinheit
Recheneinheit
14
n-te Bewertungseinheit (ωn)
Speichereinheit
Recheneinheit
15
isFEB1
isFEBn
Kombinierungseinheit $\sum_{j=0}^{n} isFEB_j \bullet \omega_j$
Speichereinheit
Recheneinheit
16
Alarmierung, wenn $\sum_{j=0}^{n} isFEB_j \bullet \omega_j > \Theta$
17

Fig.3

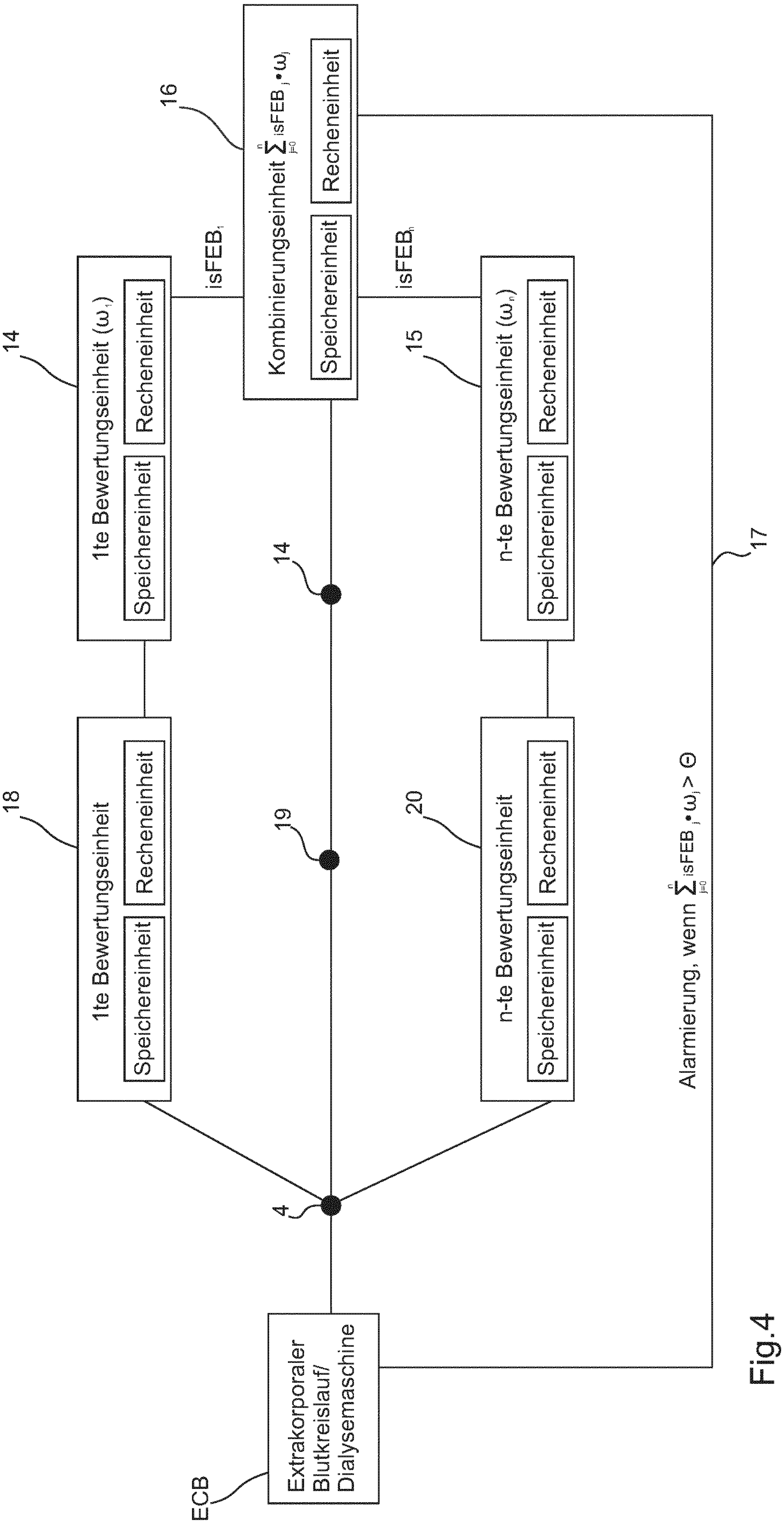

16
Kombinierungseinheit $\sum_{j=0}^{n} isFEB_j \cdot \omega_j$
Speichereinheit
Recheneinheit
$isFEB_1$
$isFEB_n$
14
1te Bewertungseinheit ($\omega_1$)
Speichereinheit
Recheneinheit
15
n-te Bewertungseinheit ($\omega_n$)
Speichereinheit
Recheneinheit
18
1te Bewertungseinheit
Speichereinheit
Recheneinheit
20
n-te Bewertungseinheit
Speichereinheit
Recheneinheit
14
19
4
17
Alarmierung, wenn $\sum_{j=0}^{n} isFEB_j \cdot \omega_j > \Theta$
ECB
Extrakorporaler Blutkreislauf/ Dialysemaschine


Fig.4

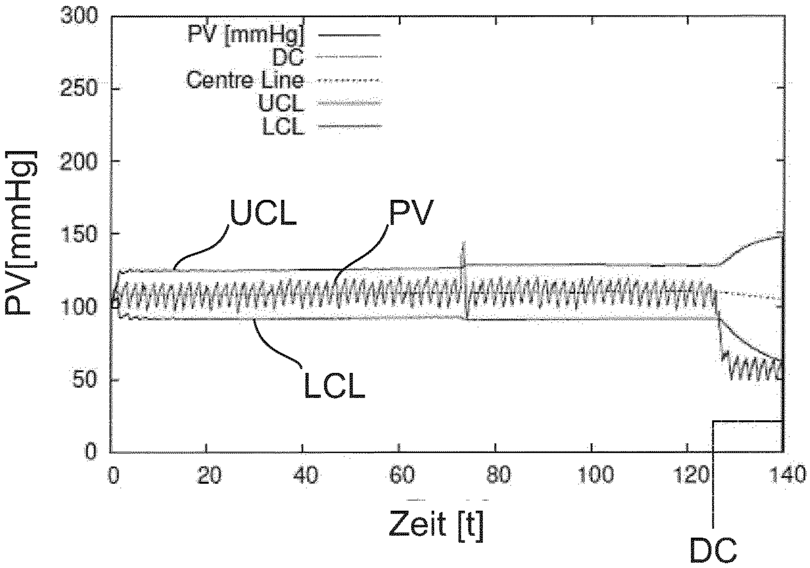
PV [mmHg]
DC
Centre Line
UCL
LCL
UCL
PV
LCL
PV[mmHg]
Zeit [t]
DC
300
250
200
150
100
50
0
0
20
40
60
80
100
120
140

Fig.5

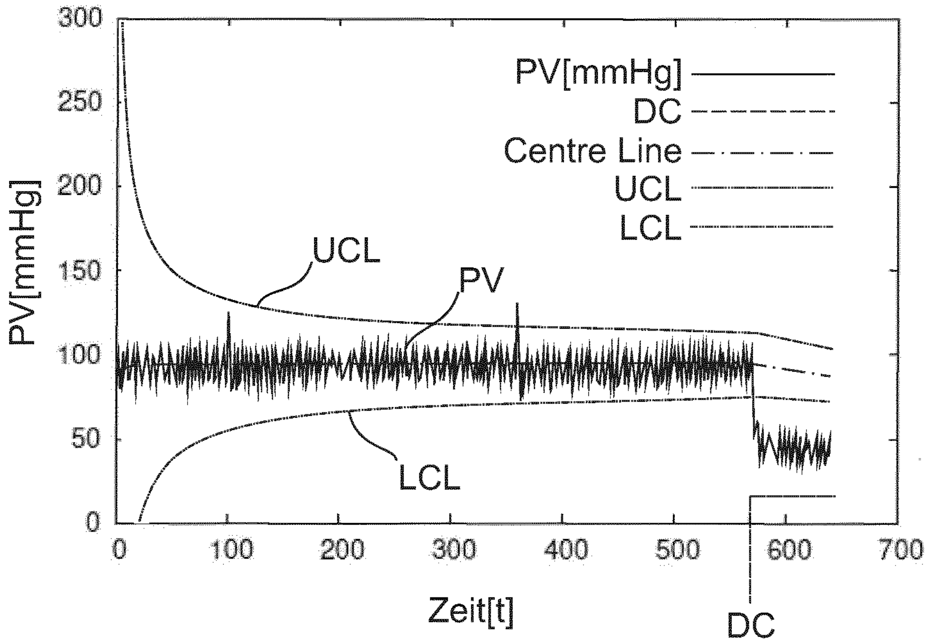
PV[mmHg]
DC
Centre Line
UCL
LCL
UCL
PV
LCL
PV[mmHg]
Zeit[t]
DC
300
250
200
150
100
50
0
0
100
200
300
400
500
600
700

Fig.6

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1584339 B1
- US 7648474 B2
- EP 1815878 B1
- DE 102012024341 B3